# FASCICULE DE BREVET EUROPEEN

(11) **EP 1 348 420 B1**
(45) Date de publication et mention de la délivrance du brevet: **09.07.2008**
(21) Numéro de dépôt: 03290530.9
(22) Date de dépôt: 05.03.2003
(51) Int. Cl.: A61K 8/60, A61K 8/73, A61K 8/81, A61K 8/90, A61Q 5/02

(54) **Composition cosmétique contenant au moins un agent tensioactif anionique, au moins un polymère cationique et au moins un copolymère acrylique séquencé, ramifié, amphiphile et procédé de traitement capillaire utilisant une telle composition**
Kosmetische Zusammensetzung, enthaltend mindestens ein anionisches Tensid, mindestens ein kationisches Polymer und mindestens ein verzweigtes, amphiphiles Acrylblockcopolymer und Haarbehandlungsverfahren unter Verwendung dieser Zusammensetzung
Cosmetic composition containing at least one anionic surfactant, at least one cationic polymer and at least one branched, amphiphilic, acrylic block copolymer and hair treatment process using said composition

(30) Priorité: 28.03.2002 FR 0203955
(43) Date de publication de la demande: 01.10.2003
(73) Titulaire: L'ORÉAL, 75008 Paris (FR)
(72) Inventeur: Maubru, Mireille, 78400 Chatou (FR)
(74) Mandataire: Le Blainvaux Bellegarde, Françoise

(56) Documents cités:
- WO-A-00/40628
- WO-A-01/05365
- WO-A-01/19946
- WO-A-01/96429
- WO-A-92/10162
- WO-A-95/34271
- FR-A- 2 798 846
- US-A- 5 391 368
- US-A- 5 656 257

## Description

La présente invention concerne une composition cosmétique contenant à la fois un agent tensioactif anionique, un polymère choisi dans un groupe particulier de polymères acryliques, à blocs, ramifiés, amphiphiles, et un agent de conditionnement choisi parmi les polymères cationiques, ainsi qu'un procédé de traitement capillaire utilisant une telle composition.

Bien que l'on connaisse dans le domaine du coiffage un très grand nombre de polymères fixants, la plupart d'entre eux présentent un pouvoir fixant limité lorsqu'ils sont utilisés dans des compositions capillaires rincées telles que des shampooings ou après-shampooings.

On a découvert récemment les propriétés de coiffage très intéressantes d'un groupe de copolymères acryliques séquencés (ou à blocs), ramifiés, amphiphiles qui présentent une structure particulière dans laquelle des blocs de monomères acryliques relativement hydrophiles sont fixés sur un bloc de monomères acryliques relativement plus hydrophobe que les blocs qu'il porte. Ces nouveaux copolymères séquencés, ramifiés, amphiphiles sont décrits dans les demandes internationales WO 00/40628 et WO 01/96429.

Ces documents décrivent non seulement la structure de ces nouveaux polymères mais également un procédé de préparation de ceux-ci ainsi que leur utilisation dans le domaine du coiffage sous forme de compositions de coiffage non rincées, telles que des laques et des mousses coiffantes sous forme de compositions aérosols contenant des solvants organiques et des agents propulseurs.

La demanderesse a découvert que les copolymères acryliques, séquencés, ramifiés, amphiphiles divulgués dans les demandes internationales citées ci-dessus, pouvaient également être utilisés dans des compositions rincées lorsqu'on les associait à au moins un polymère cationique et à un tensioactif anionique.

En effet, l'introduction, dans une base tensioactive anionique, de l'association d'au moins un polymère cationique et d'au moins un copolymère acrylique anionique, séquencé, ramifié tel que décrit ci-après, donne des shampooings qui présentent d'excellentes propriétés coiffantes et qui confèrent aux cheveux de bonnes propriétés cosmétiques, notamment en ce qui concerne le lissage et la brillance.

Les bons résultats obtenus avec l'association de polymères de la présente invention n'étaient nullement prévisibles car l'utilisation de polymères cationiques en association avec des polymères anioniques dans une base tensioactive pour apporter un effet coiffant n'a jusqu'ici pas permis l'obtention de propriétés cosmétiques satisfaisantes, notamment en ce qui concerne le lissage et la brillance des cheveux.

Les compositions de l'invention peuvent également être utilisées sur la peau. Elles permettent alors d'obtenir de très bonnes propriétés conditionnantes.

La présente invention a par conséquent pour objet une composition cosmétique comprenant, dans un milieu cosmétiquement acceptable,
- au moins un polymère amphiphile choisi parmi les copolymères à blocs, ramifiés, comprenant
   (a) des motifs non ioniques dérivés d'au moins un monomère choisi parmi les (méth)acrylates d'alkyle en C₁₋C₂₀, les N-mono-(alkyle en C₂₋C₁₂)-(méth)acrylamides et les N,N-di-(alkyle en C₂₋C₁₂)-(méth)acrylamide,
   (b) des motifs anioniques dérivés d'au moins un monomère choisi parmi l'acide acrylique et l'acide méthacrylique, et
   (c) des motifs polyfonctionnels dérivés d'au moins un monomère comprenant au moins deux groupes fonctionnels insaturés polymérisables,
   et ayant de préférence une structure constituée de blocs hydrophobes sur lesquels sont fixés, par l'intermédiaire des motifs polyfonctionnels (c), plusieurs blocs plus hydrophiles,
- au moins un polymère cationique, et
- au moins 3 % en poids, rapporté au poids total de la composition, d'au moins un agent tensioactif anionique.

L'invention a également pour objet un procédé de traitement des matières kératiniques consistant à appliquer sur lesdites matières une composition telle que décrite ci-dessus et à procéder, après un éventuel temps de pose, au rinçage des matières kératiniques.

Le temps de pose de la composition peut être compris entre 0 seconde et 30 minutes.

Les copolymères acryliques ramifiés utilisés dans la présente invention ainsi que leur préparation sont décrits dans les demandes internationales WO 00/40628 et WO 01/96429.

Ces copolymères acryliques ont une structure macromoléculaire particulière dans laquelle des séquences relativement hydrophiles, comprenant des monomères anioniques, sont fixées par l'intermédiaire de motifs polyfonctionnels (par exemple des motifs comprenant au moins deux doubles liaisons carbone-carbone polymérisables) sur des blocs relativement plus hydrophobes.

Ces copolymères sont obtenus par un procédé de polymérisation en deux étapes : dans une première étape, on fait polymériser des monomères ou un mélange de monomères, relativement hydrophobes, en présence d'un comonomère polyfonctionnel comprenant au moins deux fonctions polymérisables ayant différentes réactivités. Dans cette première étape, les monomères hydrophobes polymérisent en réagissant avec la fonction la plus réactive du comonomère polyfonctionnel de manière à former une chaîne copolymère portant un certain nombre de groupes polymérisables correspondant aux groupes moins réactifs des comonomères polyfonctionnels. Dans une deuxième étape, on fait réagir le copolymère à groupes polymérisables, obtenu dans la première étape, avec des monomères ou un mélange de monomères plus hydrophiles dont une certaine fraction porte des groupes acide carboxylique. Ces monomères plus hydrophiles polymérisent en réagissant avec les groupes polymérisables du copolymère hydrophobe de manière à former des blocs plus hydrophiles, fixés sous forme de ramifications sur ce premier copolymère.

Comme il est expliqué dans les demandes WO 00/40628 et WO 01/96429, les séquences formant ces copolymères acryliques ramifiés ne diffèrent pas seulement par leur caractère plus ou moins hydrophobe, mais également par leur température de transition vitreuse. En effet, les séquences plus hydrophiles sont des séquences dites "dures" (*hard*) car elles ont une température de transition vitreuse supérieure à la température ambiante (20 °C), alors que les séquences plus hydrophobes sont des séquences dites "souples" (*soft*) car elles ont une température de transition vitreuse bien au-dessous de la température ambiante.

Le caractère séquencé des copolymères acryliques utilisés dans la présente invention se manifeste par conséquent par l'existence d'au moins deux températures de transition vitreuse (Tg), au moins une étant supérieure et au moins une autre inférieure à la température ambiante (20 °C).

Les blocs les plus hydrophobes des copolymères acryliques séquencés ont de préférence une masse moléculaire moyenne en poids de 10 000 à 100 000, et les blocs les plus hydrophiles une masse moléculaire moyenne en poids de 1000 à 100 000.

Chacun des monomères formant les motifs polyfonctionnels (c) porte de préférence au moins deux groupes fonctionnels polymérisables qui ont une réactivité différente l'une de l'autre. Cette différence de réactivité permet de ne polymériser, dans un premier temps, que les fonctions les plus réactives et de préserver les groupes fonctionnels moins réactives qui serviront, dans un deuxième temps, à la fixation des séquences plus hydrophiles.

On peut citer à titre d'exemples de tels groupes fonctionnels polymérisables les groupes vinyle, allyle, acryloyle et méthacryloyle, ces deux derniers ayant une réactivité considérablement plus importante que les premiers.

Des motifs polyfonctionnels (c) préférés sont ceux dérivés d'un monomère correspondant à la formule suivante 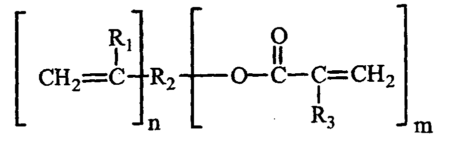 dans laquelle
n et m sont compris entre 1 et 4,
la somme de m + n est supérieure ou égale à 2,
R₁ et R₃ représente un atome d'hydrogène ou un groupe alkyle en C₁₋C₂₂, de préférence en C₁-C3,
R₂ représente un groupe alkylène en C₁₋C₂₂, cycloalkylène en C₃₋C₆, arylène en C₆₋C₁₈, alkylarylène en C₇₋C₂₄, un groupe -(CH₂-CH₂-O)ₚ où p est compris entre 1 et 50, un groupe -(CH₂(CH₃)-CH₂-O)ₚ où p est compris entre 1 et 50, ou un groupe amido, ester, polyamido, ou polyester.

Parmi ces monomères (c), on préfère tout particulièrement le méthacrylate d'allyle, l'acrylate d'allyle, le méthacrylate de vinyle, l'acrylate de vinyle, le vinylacrylamide, le vinylméthacrylamide, l'allylméthacrylamide, l'allylacrylamide et des mélanges de ceux-ci, et en particulier le méthacrylate d'allyle.

Parmi les polymères décrits ci-dessus, on préfère en particulier ceux qui comprennent essentiellement des motifs (a), (b) et (c) tels que décrits ci-dessus.

Les proportions des différents motifs (a), (b) et (c) dans le copolymère acrylique séquencé sont de préférence les suivantes :
(a) motifs anioniques : de 5 à 95 %, en particulier de 5 à 50 % en moles,
(b) motifs non ioniques : de 5 à 70 %, en particulier de 10 à 70 % en moles,
(c) motifs polyfonctionnels : de 0,005 à 2 %, en particulier de 0,1 à 1,5 % en moles.

Parmi les polymères décrits dans ces documents, on préfère un groupe de copolymères anioniques, constitués essentiellement
(a) de motifs non ioniques dérivés d'acrylate de butyle,
(b) de motifs anioniques dérivés d'acide méthacrylique et d'acide acrylique, et
(c) de motifs bifonctionnels dérivés de méthacrylate d'allyle,
et, à l'intérieur de ce groupe, en particulier les copolymères constitués de 27,5 % à 30,5 % en moles d'acrylate de butyle, de 26 % à 36 % en moles d'acide acrylique, de 33,3 % à 45,3 % en moles d'acide méthacrylique et de 0,48 % à 0,92 % en moles de méthacrylate d'allyle.

Un tel copolymère préféré est proposé sous la dénomination FIXATE® G100 par la société NOVEON.

Les compositions de la présente invention contiennent de préférence de 0,01 à 10 % en poids, et en particulier de 0,1 à 5 % en poids, rapporté au poids total de la composition, de copolymères acrylique séquencé ramifiés.

Les compositions selon l'invention comprennent, en plus d'au moins un copolymère acrylique séquencé ramifié tel que décrit ci-dessus, un ou plusieurs polymères cationiques.

Par polymère cationique, on entend tout polymère contenant des groupes cationiques et/ou des groupes ionisables en groupes cationiques.

Ces polymères cationiques sont choisis parmi tous ceux déjà connus en tant que tels pour leur capacité d'améliorer les propriétés cosmétiques des cheveux traités par des compositions détergentes. On peut citer notamment ceux décrits dans les demandes de brevet EP 0 337 354, FR 2 270 846, FR 2 383 660, FR 2 598 611, FR 2 470 596 et FR 2 519 863.

Les polymères cationiques préférés sont choisis parmi ceux qui contiennent des motifs comprenant des groupements amines primaires, secondaires, tertiaires et/ou quaternaires qui font partie de la chaîne macromoléculaire principale, ou bien sont portés par des groupes latéraux directement reliés à celle-ci.

Parmi les polymères cationiques, on peut citer plus particulièrement les polymères du type polyamine, polyaminoamide et polyammonium quaternaire. Il s'agit de produits connus.

Les polymères du type polyamine, polyaminoamide et polyammonium quaternaire, que l'on peut utiliser dans les compositions de la présente invention, sont ceux décrits dans les brevets FR 2 505 348 et FR 2 542 997. Parmi ces polymères, on peut citer en particulier :
(1) les homopolymères ou copolymères dérivés d'esters ou d'amides acryliques ou méthacryliques et comprenant au moins un des motifs de formules suivantes: 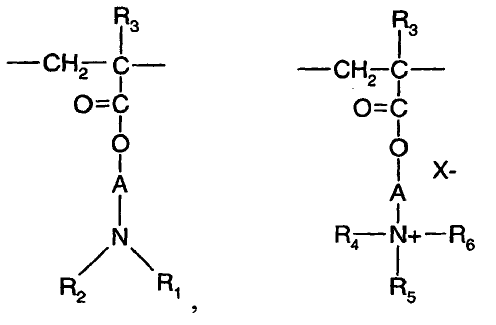 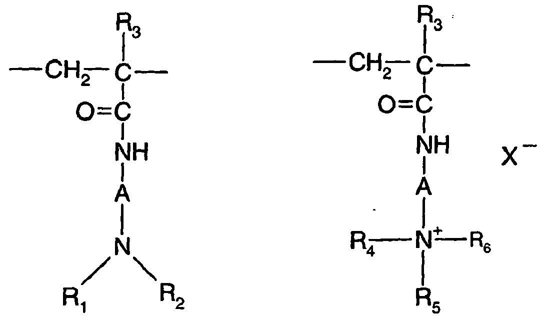 dans lesquelles:
   R₃, identiques ou différents, désignent un atome d'hydrogène ou un groupe CH₃ ;
   A, identiques ou différents, représentent un groupe alkyle, linéaire ou ramifié, comprenant de 1 à 6 atomes de carbone, de préférence 2 ou 3 atomes de carbone, ou un groupe hydroxyalkyle comprenant de 1 à 4 atomes de carbone ;
   R₄, R₅, R₆, identiques ou différents, représentent un groupe alkyle ayant de 1 à 18 atomes de carbone ou un groupe benzyle, et de préférence un groupe alkyle ayant de 1 à 6 atomes de carbone;
   R₁ et R₂, identiques ou différents, représentent un atome d'hydrogène ou un groupe alkyle ayant de 1 à 6 atomes de carbone, et de préférence un groupe méthyle ou éthyle;
   X⁻ désigne un anion dérivé d'un acide minéral ou organique tel qu'un anion méthosulfate ou un halogénure comme le chlorure ou le bromure.
   Les copolymères de la famille (1) peuvent contenir en outre un ou plusieurs motifs dérivant de comonomères choisis dans la famille des acrylamides, méthacrylamides, diacétone-acrylamides, acrylamides et méthacrylamides substitués sur l'atome d'azote par des groupes alkyle inférieur en C₁₋₄, des groupes dérivés des acides acryliques ou méthacryliques ou de leurs esters, de vinyllactames tels que la vinylpyrrolidone ou le vinylcaprolactame, d'esters vinyliques.
   Parmi ces copolymères de la famille (1), on peut citer en particulier :
   - les copolymères d'acrylamide et de méthacrylate de diméthylaminoéthyle quaternisé au sulfate de diméthyle ou avec un halogénure de diméthyle, tels que celui vendu sous la dénomination HERCOFLOC® par la société HERCULES,
   - les copolymères d'acrylamide et de chlorure de méthacryloyloxyéthyltriméthylammonium décrits, par exemple, dans la demande de brevet EP-A-080976 et vendus sous la dénomination BINAQUAT® P 100 par la société CIBA GEIGY,
   - le copolymère d'acrylamide et de méthosulfate de méthacryloyloxyéthyltriméthylammonium vendu sous la dénomination RETEN® par la société HERCULES,
   - les copolymères vinylpyrrolidone/acrylate ou méthacrylate de dialkylaminoalkyle quaternisés ou non, tels que les produits vendus sous la dénomination GAFQUAT® par la société ISP comme, par exemple, GAFQUAT® 734 ou GAFQUAT® 755, ou bien les produits dénommés COPOLYMER 845, 958 et 937. Ces polymères sont décrits en détail dans les brevets FR 2 077 143 et FR 2 393 573,
   - les terpolymères méthacrylate de diméthylaminoéthyle/vinylcaprolactame/vinylpyrrolidone tels que le produit vendu sous la dénomination GAFFIX® VC 713 par la société ISP,
   - les copolymères vinylpyrrolidone/méthacrylamidopropyl-diméthylamine commercialisés notamment sous la dénomination STYLEZE® CC 10 par ISP, et
   - les copolymères vinylpyrrolidone/méthacrylamide de diméthylaminopropyle quaternisé, tels que le produit vendu sous la dénomination GAFQUAT® HS 100 par la société ISP.
(2) Les dérivés d'éthers de cellulose comprenant des groupements ammonium quaternaires décrits dans le brevet FR 1 492 597, et en particulier les polymères commercialisés sous les dénominations "JR" (JR 400, JR 125, JR 30M) ou "LR" (LR 400, LR 30M) par la Société AMERCHOL. Ces polymères sont également définis dans le dictionnaire CTFA comme des ammoniums quaternaires d'hydroxyéthylcellulose ayant réagi avec un époxyde substitué par un groupement triméthylammonium.
(3) Les dérivés cationiques de la cellulose tels que les copolymères de cellulose ou les dérivés de cellulose greffés avec un monomère hydrosoluble d'ammonium quaternaire, et décrits notamment dans le brevet US 4 131 576, tels que les hydroxyalkylcelluloses, comme les hydroxyméthyl-, hydroxyéthyl- ou hydroxypropylcelluloses greffées notamment avec un sel de méthacryloyléthyl-triméthylammonium, de méthacrylamidopropyltriméthylammonium, de diméthyldiallylammonium.
   Les produits commercialisés répondant à cette définition sont plus particulièrement les produits vendus sous la dénomination Celquat® L 200 et Celquat® H 100 par la Société National Starch.
(4) Les polysaccharides cationiques décrits plus particulièrement dans les brevets US 3 589 578 et US 4 031 307, tels que les gommes de guar contenant des groupements cationiques trialkylammonium. On utilise, par exemple, des gommes de guar modifiées par un sel, par exemple le chlorure, de 2,3-époxypropyltriméthylammonium.
   De tels produits sont commercialisés notamment sous les dénominations commerciales de JAGUAR® C13 S, JAGUAR® C 15, JAGUAR® C 17 ou JAGUAR® C162 par la société RHODIA.
(5) Les polymères constitués de motifs pipérazinyle et de groupes divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets FR 2 162 025 et FR 2 280 361.
(6) Les polyaminoamides solubles dans l'eau, préparés en particulier par polycondensation d'un composé acide avec une polyamine. Ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épihalohydrine, d'un diépoxyde ou d'un dérivé bis-insaturé, l'agent réticulant étant utilisé dans des proportions allant de 0,025 à 0,35 mole par groupement amine du polyaminoamide. Ces polyaminoamides peuvent être alkylés ou, s'ils comportent une ou plusieurs fonctions amines tertiaires, quatemisées. De tels polymères sont notamment décrits dans les brevets FR 2 252 840 et FR 2 368 508.
(7) Les dérivés de polyaminoamides résultant de la condensation de polyalkylènes-polyamines avec des acides polycarboxyliques, suivie d'une alkylation par des agents bifonctionnels. On peut citer, par exemple, les polymères acide adipique/diakylaminohydroxyalkyl-dialkylènetriamine dans lesquels le groupe alkyle comporte de 1 à 4 atomes de carbone et désigne de préférence un groupe méthyle, éthyle ou propyle, et le groupe alkylène comporte de 1 à 4 atomes de carbone, et désigne de préférence le groupe éthylène. De tels polymères sont notamment décrits dans le brevet FR 1 583 363.
   Parmi ces dérivés, on peut citer plus particulièrement les polymères acide adipique/diméthylaminohydroxypropyl-diéthylène-triamine vendus sous la dénomination Cartaretine® F, F4 ou F8 par la société Sandoz.
(8) Les polymères obtenus par réaction d'une polyalkylène-polyamine comprenant deux groupements amine primaire et au moins un groupement amine secondaire, avec un acide dicarboxylique choisi parmi l'acide diglycolique et les acides dicarboxyliques aliphatiques saturés ayant de 3 à 8 atomes de carbone, le rapport molaire de la polyalkylène-polylamine à l'acide dicarboxylique étant compris entre 0,8:1 et 1,4:1. Le polyaminoamide résultant de cette réaction est ensuite amené à réagir avec l'épichlorhydrine dans un rapport molaire d'épichlorhydrine par rapport au groupement amine secondaire du polyaminoamide compris entre 0,5:1 et 1,8:1. De tels polymères sont notamment décrits dans les brevets US 3 227 615 et US 2 961 347.
   Des polymères de ce type sont en particulier commercialisés sous la dénomination Hercosett® 57 par la société Hercules Inc. ou bien sous la dénomination de PD 170 ou Delsette® 101 par la société Hercules dans le cas du copolymère d'acide adipique/époxypropyl-diéthylène-triamine.
(9) Les cyclopolymères d'alkyldiallylamine ou de dialkyldiallylammonium tels que les homopolymères ou copolymères comprenant, comme constituant principal de la chaîne, des motifs répondant aux formules (Va) ou (Vb) : 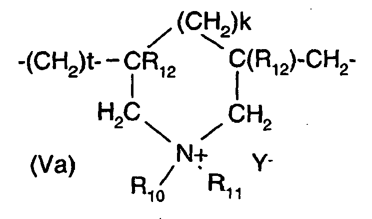 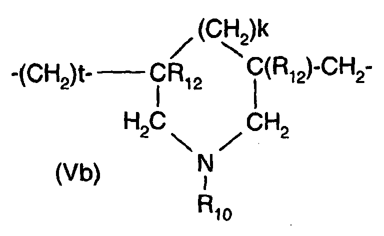 dans lesquelles
   k et t sont égaux à 0 ou 1, la somme k + t étant égale à 1 ;
   R₁₂ désigne un atome d'hydrogène ou un groupe méthyle ;
   R₁₀ et R₁₁, indépendamment l'un de l'autre, désignent un groupement alkyle ayant de 1 à 22 atomes de carbone, un groupement hydroxyalkyle en C₁₋₅, un groupement amidoalkyle inférieur en C₁-C₄, ou bien R₁₀ et R₁₁ peuvent désigner conjointement avec l'atome d'azote auquel ils sont rattachés, des groupements hétérocycliques, tels que pipéridinyle ou morpholinyle ;
   Y⁻ est un anion tel que bromure, chlorure, acétate, borate, citrate, tartrate, bisulfate, bisulfite, sulfate, phosphate.
   Ces polymères sont notamment décrits dans le brevet FR 2 080 759 et dans son certificat d'addition 2 190 406.
   Parmi les polymères définis ci-dessus, on peut citer plus particulièrement l'homopolymère de chlorure de diméthyldiallyl-ammonium vendu sous la dénomination MERQUAT® 100 par la société NALCO (et ses homologues de faibles masses moléculaires moyenne en poids) et les copolymères de chlorure de diallyldiméthylammonium et d'acrylamide commercialisés sous la dénomination MERQUAT® 550.
(10) Les polymères de diammonium quaternaire contenant des motifs récurrents répondant à la formule (VI) : 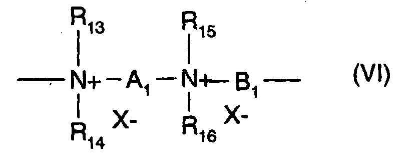 dans laquelle :
   R₁₃, R₁₄, R₁₅ et R₁₆, identiques ou différents, représentent des groupes aliphatiques, alicycliques ou arylaliphatiques contenant de 1 à 20 atomes de carbone ou des groupes hydroxyalkyle aliphatiques inférieurs, ou bien R₁₃, R₁₄, R₁₅ et R₁₆, ensemble ou séparément, forment avec les atomes d'azote auxquels ils sont rattachés des hétérocycles contenant éventuellement un second hétéroatome autre que l'azote, ou bien R₁₃, R₁₄, R₁₅ et R₁₆ représentent un groupe alkyle en C₁₋₆, linéaire ou ramifié, substitué par un groupement nitrile, ester, acyle, amide ou -CO-O-R₁₇-D ou -CO-NH-R₁₇-D où R₁₇ est un groupe alkylène et D un groupement ammonium quaternaire ;
   A₁ et B₁ représentent des groupements polyméthyléniques contenant de 2 à 20 atomes de carbone, pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A₁, R₁₃ et R₁₅ peuvent former avec les deux atomes d'azote auxquels ils sont rattachés un cycle pipérazinique ; en outre, si A₁ désigne un groupe alkylène ou hydroxyalkylène linéaire ou ramifié, saturé ou insaturé, B₁ peut également désigner un groupement :

      -(CH₂)ₙ-CO-D-OC-(CH₂)ₙ-

      n varie de 1 à 6
      dans lequel D désigne :
      a) un reste de glycol de formule -O-Z-O-, où Z désigne un groupe hydrocarboné linéaire ou ramifié, ou un groupement répondant à l'une des formules suivantes :

         -(CH₂-CH₂-O)ₓ-CH₂-CH₂-

         -[CH₂-CH(CH₃)-O]_{y}-CH₂-CH(CH₃)-

         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule -NH-Y-NH-, où Y désigne un groupe hydrocarboné linéaire ou ramifié, ou bien le groupe divalent -CH₂-CH₂-S-S-CH₂-CH₂- ;
      d) un groupement uréylène de formule -NH-CO-NH- .
      De préférence, X⁻ est un anion tel que le chlorure ou le bromure.
      Ces polymères ont une masse moléculaire moyenne en nombre généralement comprise entre 1000 et 100000.
      Des polymères de ce type sont notamment décrits dans les brevets FR 2 320 330, FR 2 270 846, FR 2 316 271, FR 2 336 434 et FR 2 413 907 et les brevets US 2 273 780, US 2 375 853, US 2 388 614, US 2 454 547, US 3 206 462, US 2 261 002, US 2 271 378, US 3 874 870, US 4 001 432, US 3 929 990, US 3 966 904, US 4 005 193, US 4 025 617, US 4 025 627, US 4 025 653, US 4 026 945 et US 4 027 020.
      On peut utiliser plus particulièrement les polymères qui sont constitués de motifs récurrents répondant à la formule : 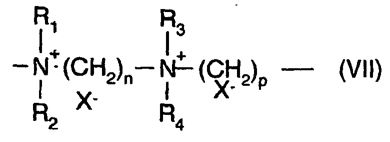 dans laquelle R₁, R₂, R₃ et R₄, identiques ou différents, désignent un groupe alkyle ou hydroxyalkyle ayant de 1 à 4 atomes de carbone environ, n et p sont des nombres entiers variant de 2 à 20 environ et, X⁻ est un anion dérivé d'un acide minéral ou organique.
      Un composé de formule (VII) particulièrement préféré est celui pour lequel R₁, R₂, R₃ et R₄ représentent un groupe méthyle et n=3, p=6 et X=Cl, dénommé chlorure d'hexadiméthrine (CTFA).
(11) Les polymères de polyammonium quaternaire, constitués de motifs de formule (VIII) : 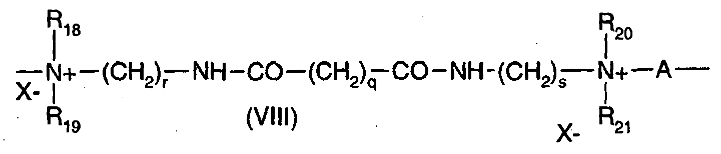 dans laquelle :
   R₁₈, R₁₉, R₂₀ et R₂₁, identiques ou différents, représentent un atome d'hydrogène ou un groupe méthyle, éthyle, propyle, β-hydroxyéthyle, β-hydroxypropyle ou -CH₂CH₂(OCH₂CH₂)ₚOH, où p est égal à 0 ou à un nombre entier compris entre 1 et 6, sous réserve que R₁₈, R₁₉, R₂₀ et R₂₁ ne représentent pas simultanément un atome d'hydrogène,
   r et s, identiques ou différents, sont des nombres entiers compris entre 1 et 6,
   q est égal à 0 ou à un nombre entier compris entre 1 et 34,
   X⁻ désigne un anion tel qu'un halogénure,
   A désigne un radical d'un dihalogénure ou représente de préférence -CH₂-CH₂-O-CH₂-CH₂-.
   De tels composés sont notamment décrits dans la demande de brevet EP-A-122 324.
   On peut citer parmi ceux-ci, par exemple, les produits Mirapol® A 15, Mirapol® AD1, Mirapol® AZ1 et Mirapol® 175 vendus par la société Miranol.
(12) Les polymères quaternaires de vinylpyrrolidone et de vinylimidazole tels que, par exemple, les produits commercialisés sous les dénominations Luviquat® FC 905, FC 550 et FC 370 par la société B.A.S.F. On peut citer notamment les copolymères de vinylpyrrolidone et de chlorure de méthylvinylimidazolium.
(13) Les polyamines comme le Polyquart® H vendu par COGNIS, référencées sous le nom de POLYETHYLENEGLYCOL (15) TALLOW POLYAMINE dans le dictionnaire CTFA.
(14) Les polymères réticulés ou non réticulés de sels de méthacryloyloxyalkyl(C₁₋₄) trialkyl(C₁₋₄)ammonium, tels que les polymères obtenus par homopolymérisation du méthacrylate de diméthylaminoéthyle quaternisé par le chlorure de méthyle, ou par copolymérisation de l'acrylamide et de méthacrylate de diméthylaminoéthyle quaternisé par le chlorure de méthyle, l'homopolymérisation ou la copolymérisation étant suivie d'une réticulation par un composé à insaturation oléfinique, en particulier le méthylène-bisacrylamide. On peut plus particulièrement utiliser un copolymère réticulé acrylamide/chlorure de méthacryloyloxy-éthyl-triméthylammonium (20/80 en poids) sous forme de dispersion contenant 50 % en poids dudit copolymère dans de l'huile minérale. Cette dispersion est commercialisée sous le nom de SALCARE® SC 92 par la Société CIBA. On peut également utiliser un homopolymère réticulé du chlorure de méthacryloyloxyéthyl-triméthylammonium contenant environ 50 % en poids de l'homopolymère dans de l'huile minérale ou dans un ester liquide. Ces dispersions sont commercialisées sous les noms de SALCARE® SC 95 et SALCARE® SC 96 par la Société CIBA.

D'autres polymères cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés cationiques de la chitine.

Parmi tous les polymères cationiques susceptibles d'être utilisés dans les compositions cosmétiques de la présente invention, on préfère les dérivés d'éthers de cellulose comprenant des groupements ammonium quaternaires, tels que les produits vendus sous la dénomination JR 400 par la Société AMERCHOL, les cyclopolymères cationiques, en particulier les homopolymères ou copolymères de chlorure de diméthyldiallylammonium, vendus sous les dénominations MERQUAT® 100, MERQUAT® 550 et MERQUAT® S par la société NALCO, les polysaccharides cationiques tels que les gommes de guar modifiées par un sel de 2,3-époxypropyltriméthylammonium, les copolymères quaternisés de vinylpyrrolidone et de vinylimidazole, les polycondensats de polyammonium quaternaire comprenant de préférence les motifs récurrents de formules (VI) et (VIII) telles qu'indiquées ci-dessus, et leurs mélanges.

Encore plus préférentiellement, on utilisera les copolymères quaternisés de vinylpyrrolidone et de vinylimidazole.

La concentration des copolymères cationiques dans les compositions de la présente invention est de préférence comprise entre 0,001 % et 10 % en poids, plus préférentiellement entre 0,05 et 5 % en poids et en particulier entre 0,05 et 1 % en poids, rapportée au poids total de la composition.

Comme agents tensioactifs anioniques utilisables dans la présente invention, on peut notamment mentionner les sels, en particulier les sels de métaux alcalins tels que les sels de sodium, les sels d'ammonium, les sels d'amines, les sels d'aminoalcools ou les sels de métaux alcalino-terreux, par exemple, de magnésium, des types suivants : les alkylsulfates, les alkyléthersulfates, les alkylamidoéthersulfates, les alkylarylpolyéthersulfates, les monoglycéride-sulfates ; les alkylsulfonates, les alkylamidesulfonates, les alkylarylsulfonates, les α-oléfine-sulfonates, les paraffine-sulfonates, les alkylsulfosuccinates, les alkyléthersulfosuccinates, les alkylamidesulfosuccinates, les alkylsulfo-acétates, les acylsarcosinates et les acylglutamates, les groupes alkyle et acyle de tous ces composés comprenant de 6 à 24 atomes de carbone et le groupe aryle désignant de préférence un groupe phényle ou benzyle.

On peut également utiliser les monoesters d'alkyle en C₆₋C₂₄ et d'acides polyglycoside-dicarboxyliques tels que les glucoside-citrates d'alkyle, les polyglycoside-tartrates d'alkyle et les polyglycoside-sulfosuccinates d'alkyle, les alkylsulfosuccinamates, les acyliséthionates et les N-acyltaurates, le groupe alkyle ou acyle de tous ces composés comprenant de 12 à 20 atomes de carbone.

Un autre groupe d'agents tensioactifs utilisables dans les compositions de la présente invention est celui des acyl-lactylates dont le groupe acyle comporte de 8 à 20 atomes de carbone.

En outre, on peut encore citer les acides alkyl-D-galactoside uroniques et leurs sels ainsi que les acides alkyl(C₆₋C₂₄)éther-carboxyliques polyoxyalkylénés, les acides alkyl(C₆₋C₂₄)aryl(C₆₋C₂₄)éther-carboxyliques polyoxyalkylénés, les acides alkyl(C₆₋C₂₄)amidoéther carboxyliques polyoxyalkylénés et leurs sels, en particulier ceux comprenant de 2 à 50 motifs oxyde d'éthylène, et leurs mélanges.

On utilise de préférence les alkylsulfates, les alkyléthersulfates et les alkyléthercarboxylates, et leurs mélanges, en particulier sous forme de sels de métaux alcalins ou alcalino-terreux, d'ammonium, d'amine ou d'aminoalcool.

On peut éventuellement introduire dans les compositions de l'invention un ou plusieurs agents tensioactifs amphotères ou non ioniques.

Les agents tensioactifs amphotères, utilisables dans la présente invention, peuvent être notamment des dérivés d'amines aliphatiques secondaires ou tertiaires, dans lesquels le groupe aliphatique est une chaîne linéaire ou ramifiée comprenant de 8 à 22 atomes de carbone et contenant au moins un groupe anionique tel que, par exemple, un groupe carboxylate, sulfonate, sulfate, phosphate ou phosphonate. On peut citer en particulier les alkyl(C₈₋C₂₀)bétaïnes, les sulfobétaïnes, les alkyl(C₈₋C₂₀)amidoalkyl(C2₋C₈)-bétaïnes ou les alkyl(C₈₋C₂₀)amidoalkyl(C₂₋C₈)sulfobétaïnes.

Parmi les dérivés d'amines, on peut citer les produits commercialisés sous la dénomination MIRANOL®, tels que décrits dans les brevets US 2 528 378 et US 2 781 354 et classés dans le dictionnaire CTFA, 3^{ème} édition, 1982, sous les dénominations Amphocarboxy-glycinate et Amphocarboxypropionate de structures respectives (1) et (2) :

Rₐ-CONHCH₂CH₂-N(R_{b})(R_{c})(CH₂COO⁻) (1)

dans laquelle :
Rₐ représente un groupe alkyle dérivé d'un acide Rₐ-COOH présent dans l'huile de coprah hydrolysée, un groupe heptyle, nonyle ou undécyle,
R_{b} représente un groupe bêta-hydroxyéthyle, et
R_{c} représente un groupe carboxyméthyle ;
et

   Rₐ'-CONHCH₂CH₂-N(B)(C) (2)
dans laquelle :
B représente -CH₂CH₂OX',
C représente -(CH₂)_{z}-Y', avec z = 1 ou 2,
X' représente le groupe -CH₂CH₂-COOH ou un atome d'hydrogène,
Y' représente -COOH ou le groupe -CH₂-CHOH-SO₃H,
Rₐ' représente un groupe alkyle d'un acide Rₐ'-COOH présent dans l'huile de coprah ou dans l'huile de lin hydrolysée, un groupe alkyle, notamment en C₁₇ et sa forme iso, un groupe en C₁₇ insaturé.

Ces composés sont classés dans le dictionnaire CTFA, 5^{ème} édition, 1993, sous les dénominations cocoamphodiacétate de disodium, lauroamphodiacétate de disodium, caprylamphodiacétate de disodium, capryloamphodiacétate de disodium, cocoamphodipropionate de disodium, lauroamphodipropionate de disodium, caprylamphodipropionate de disodium, capryloamphodipropionate de disodium, acide lauroamphodipropionique, acide cocoamphodipropionique.

A titre d'exemple, on peut citer le cocoamphodiacétate commercialisé par la société RHODIA sous la dénomination commerciale MIRANOL® C2M concentré.

Parmi les tensioactifs amphotères, on utilise de préférence les (alkyle en C₈₋C₂₀)-bétaïnes, les (alkyle en C₈₋C₂₀)-amido(alkyle en C₂₋C₈)bétaïnes, les alkylamphodiacétates et leurs mélanges.

Les tensioactifs non-ioniques utilisables dans les compositions de la présente invention sont des composés bien connus en soi (voir notamment à cet égard "Handbook of Surfactants" par M.R. PORTER, éditions Blackie & Son (Glasgow and London), 1991, pp 116-178). Ils sont choisis notamment parmi les alcools, les alpha-diols, les alkyl(C₁₋₂₀)phénols ou les acides gras polyéthoxylés, polypropoxylés ou polyglycérolés, ayant une chaîne grasse comprenant, par exemple, de 8 à 18 atomes de carbone, le nombre de groupements oxyde d'éthylène ou oxyde de propylène pouvant aller notamment de 2 à 50 et le nombre de groupements glycérol pouvant aller notamment de 2 à 30.

On peut également citer les copolymères d'oxyde d'éthylène et de propylène, les condensats d'oxyde d'éthylène et de propylène sur des alcools gras ; les amides gras polyéthoxylés ayant de préférence de 2 à 30 motifs d'oxyde d'éthylène, les amides gras polyglycérolés comprenant en moyenne de 1 à 5 groupements glycérol et en particulier de 1,5 à 4, les esters d'acides gras du sorbitane éthoxylés ayant de 2 à 30 motifs d'oxyde d'éthylène, les esters d'acides gras du saccharose, les esters d'acides gras du polyéthylèneglycol, les (alkyle en C₆₋C₂₄)polyglycosides, les dérivés de N-(alkyle en C₆₋C₂₄)glucamine, les oxydes d'amines tels que les oxydes d'(alkyle en C₁₀₋C₁₄)amines ou les oxydes de N-(acyle en C₁₀₋C₁₄)-aminopropylmorpholine.

Parmi les tensioactifs non-ioniques cités ci-dessus, on utilise de préférence les (alkyle en C₆₋C₂₄)polyglycosides.

Comme indiqué ci-dessus, la quantité d'agents tensioactifs anioniques est au moins égale à 3 % en poids, rapportée au poids total de la composition cosmétique. Elle est de préférence comprise entre 5 % et 35 % en poids et, mieux encore, entre 8 % et 25 % en poids.

La quantité totale d'agents tensioactifs amphotères et/ou non ioniques, lorsqu'ils sont présents, est de préférence comprise entre 0,5 et 30 %, et en particulier entre 1 et 20 % rapportée au poids total de la composition.

Le milieu aqueux cosmétiquement acceptable peut être constitué uniquement d'eau ou d'un mélange d'eau et d'un ou de plusieurs solvants cosmétiquement acceptables tels que les alcools inférieurs en C₁-C₄, en particulier l'éthanol, l'isopropanol, le tertio-butanol et le n-butanol, les alkylèneglycols comme le propylèneglycol, les éthers de polyol, les alcanes en C₅-C₁₀, l'acétone, la méthyléthylcétone, les acétates d'alkyle en C₁-C₄ comme l'acétate de méthyle, l'acétate d'éthyle et l'acétate de butyle, le diméthoxyéthane et le diéthoxyéthane.

Le pH des compositions de la présente invention est de préférence compris entre 3 et 8, et en particulier entre 4 et 7.

Les compositions selon l'invention peuvent également contenir des additifs de formulation tels que des épaississants polymériques naturels ou synthétiques, anioniques, amphotères, zwittérioniques, non ioniques ou cationiques, associatifs ou non, des épaississants non polymériques comme des acides ou des électrolytes, des agents nacrants, des agents opacifiants, des parfums, des huiles minérales, végétales et/ou synthétiques, des esters d'acides gras, des silicones volatiles ou non volatiles, organomodifiées ou non, des colorants, des agents conservateurs ou des agents de stabilisation du pH.

L'homme de métier veillera à choisir les éventuels additifs et leur quantité de manière à ce qu'ils ne nuisent pas aux propriétés intéressantes des compositions de la présente invention.

Ces additifs sont présents dans la composition selon l'invention en une quantité totale allant de 0 à 20 % en poids par rapport au poids total de la composition.

Les compositions de la présente invention peuvent être des solutions homogènes, des suspensions, des émulsions eau-dans-huile (E/H), des émulsions huile-dans-eau (H/E) ou des émulsions multiples, toutes ayant une consistance fluide, plus ou moins épaissie, ou bien gélifiée.

De préférence, les compositions de la présente invention sont conditionnées en l'absence d'agent propulseurs.

Les compositions de l'invention peuvent se présenter sous forme de shampooings, de produits pour le bain ou pour la douche, ou de produit démaquillant. La composition de l'invention est de préférence un shampooing.

L'exemple suivant illustre la présente invention.

### Exemple 1

On a préparé les compositions de shampooing suivantes:

| | |
|---|---|
| Lauryléthersulfate de sodium (C₁₂/C₁₄ à 70/30) à 2,2 moles d'oxyde d'éthylène | 10,5 g (m.a.)^{a)} |
| Alkyl(C₈/C₁₆)polyglycoside (PLANTAREN® 2000, de COGNIS) | 4,0 g (m.a) |
| Alcool laurylique oxyéthyléné (2,5 motifs OE) | 1,0 g |
| Copolymère de vinylpyrrolidone et de chlorure de méthylvinylimidazolium (5/95) en solution aqueuse à 40 % (LUVIQUAT® FC 905 (BASF)) | 0,1 g (m.a.) |
| Copolymère à blocs, ramifié, amphiphile d'acrylate de butyle, d'acide acrylique, d'acide méthacrylique et de méthacrylate d'allyle en solution aqueuse à 27 % en poids (FIXATE® G100 (société NOVEON) | 0,2 g (m.a.) |
| Poly(acide acrylique) réticulé | 0,2 g |
| Distéaryléther | 1,5 g |
| Mélange d'alcools gras à 76 % d'alcool béhénylique (NAFOL® 1822 C (société CONDEA)) | 1,5 g |
| Acide citrique | q.s.p. pH 5 |
| Chlorure de sodium | 1,5 g |
| Parfum, conservateurs | q.s. |
| Eau déminéralisée | q.s.p. 100 g |

| | |
|---|---|
| ^{a)} m.a. = matière active | |

Ce shampooing présente de bonnes propriété coiffantes (gonflant, tenue) et cosmétiques, notamment en ce qui concerne le lissage et la brillance.

### Exemples 2 et 3

On a préparé les compositions de shampooing suivantes:

| Composition | Exemple 2 | Exemple 3 |
|---|---|---|
| Lauryléthersulfate de sodium (C12/C14 à 70/30) à 2.2 moles d'oxyde d'éthylène | 14 g M.A. | 10 g M.A. |
| Lauryléthersulfate de sodium / magnésium (80/20) à 4 moles d'oxyde d'éthylène | - | 3,1 g M.A. |
| Cocoamidopropyl bétaïne | 2,4 g M.A. | - |
| Cocoamidoéthyl (N-hydroxyéthyl, N-carboxyméthyl) glycinate de sodium, vendu sous la dénomination Miranol C2M par la société RHODIA | - | 4,5 g M.A. |
| Chlorure d'hydroxypropylguar triméthylammonium, vendu sous la dénomination Jaguar C-162 par la société RHODIA | - | 0,25 g |
| Hydroxyéthylcellulose réticulée à l'épichlorhydrine, quaternisée par la triméthylamine, vendue sous la dénomination JR 400 par la société AMERCHOL | 0,4 g | - |
| Polymère séquencé branché acrylate de butyle / acide acrylique / acide méthacrylique en solution aqueuse à 27% M.A. proposé sous la dénomination FIXATE G100 par la société NOVEON | 3,75 g | 3,75 g |
| Polydiméthylsiloxane de viscosité 500 000 cst, vendue sous la dénomination Mirasil DM 500 000 par la société RHODIA | 1,5 g | - |
| Acide polyacrylique réticulé | 0,2 g | 0,1 g |
| Monoisopropanolamide d'acides de coprah | 1,2 g | 0,2 g |
| Alcool béhénylique | 1,5 g | - |
| Distéaryléther | 1,5 g | |
| Distéarate d'éthylèneglycol | - | 2 g |
| Hydroxyde de sodium q.s.. | pH 6.5 | |
| Acide citrique q.s. | - | pH 6 |
| Parfum, conservateurs | q.s. | q.s. |
| Eau déminéraliséeq.s.p. | 100 g | 100 g |

Ces compositions présentent de bonnes propriétés coiffantes (gonflant, tenue) et cosmétiques, notamment sur les critères de démêlage et de brillance.

## Revendications

1. Composition cosmétique comprenant, dans un milieu cosmétiquement acceptable,
• au moins un polymère amphiphile choisi parmi les copolymères à blocs, ramifiés, comprenant
(a) des motifs non ioniques dérivés d'au moins un monomère choisi parmi les (méth)acrylates d'alkyle en C₁-C₂₀, les N-mono-(alkyle en C₂-C₁₂)-(méth)acrylamides et les N,N-di-(alkyle en C₂-C₁₂)-(méth)acrylamide,
(b) des motifs anioniques dérivés d'au moins un monomère choisi parmi l'acide acrylique et l'acide méthacrylique, et
(c) des motifs polyfonctionnels dérivés d'au moins un monomère comprenant au moins deux groupes fonctionnels insaturés polymérisables,

2. Composition selon la revendication 1, **caractérisé par le fait que** le polymère amphiphile choisi parmi les copolymères à blocs, ramifiés, a une structure constituée de blocs hydrophobes sur lesquels sont fixés, par intermédiaire des motifs polyfonctionnels (c), plusieurs blocs plus hydrophiles,
• au moins un polymère cationique, et
• au moins 3 % en poids, rapporté au poids total de la composition, d'au moins un agent tensioactif anionique.

3. Composition selon la revendication 1 ou 2, **caractérisée par le fait que** le polymère amphiphile présente au moins deux températures de transition vitreuse (Tg) dont au moins une est supérieure à la température ambiante (20 °C) et l'autre est inférieure à la température ambiante.

4. Composition selon l'une des revendications 1 à 3, **caractérisée par le fait que** le polymère amphiphile est un copolymère anionique comprenant essentiellement
(a) des motifs non ioniques dérivés d'acrylate de butyle,
(b) des motifs anioniques dérivés d'acide méthacrylique et d'acide acrylique, et
(c) des motifs polyfonctionnels dérivés de méthacrylate d'allyle.

5. Composition selon la revendication 4, **caractérisée par le fait que** le polymère amphiphile est constitué essentiellement
(a) de 27,5 % à 30,5 % en moles d'acrylate de butyle,
(b) de 26 % à 36 % en moles d'acide acrylique et de 33,3 % à 45,3 % en moles d'acide méthacrylique, et
(c) de 0,48 à 0,92 % en moles de méthacrylate d'allyle.

6. Composition selon l'une quelconque des revendications précédentes, **caractérisée par le fait que** la concentration du polymère amphiphile est comprise entre 0,01 % et 10 % en poids, rapportée au poids total de la composition.

7. Composition selon l'une quelconque des revendications précédentes, **caractérisée par le fait que** le ou les polymères cationiques sont choisis parmi les éthers de cellulose comprenant des groupements ammonium quaternaires, les cyclopolymères cationiques à base de chlorure de diméthyldiallylammonium, les polysaccharides cationiques tels que les gommes de guar modifiées par un sel de 2,3-époxypropyl-triméthyl-ammonium, les polymères quaternisés de vinylpyrrolidone et de vinylimidazole et les polycondensats de polyammonium quaternaire.

8. Composition selon l'une quelconque des revendications précédentes, **caractérisée par le fait que** le ou les polymères cationiques sont présents en une quantité comprise entre 0,001 % et 10 % en poids rapportée au poids total de la composition.

9. Composition selon l'une quelconque des revendications précédentes, **caractérisée par le fait que** le ou les agents tensioactifs anioniques sont choisis parmi les alkylsulfates, les alkyléthersulfates et les alkyléthercarboxylates, et leurs mélanges.

10. Composition selon l'une quelconque des revendications précédentes, **caractérisée par le fait que** la concentration d'agents tensioactifs anioniques est comprise entre 5 et 35 % en poids rapportée au poids total de la composition.

11. Composition selon l'une quelconque des revendications précédentes, **caractérisée par le fait qu'**elle contient en outre au moins un agent tensioactif choisi parmi les agents tensioactifs non-ioniques et les agents tensioactifs amphotères.

12. Composition selon la revendication 11, **caractérisée par le fait que** l'agent tensioactif non-ionique est un (alkyle en C₆₋C₂₄)-polyglycoside.

13. Composition selon la revendication 11, **caractérisée par le fait que** l'agent tensioactif amphotère est choisi parmi les (alkyle en C₈₋C₂₀)-bétaïnes, les (alkyle en C₈-C₂₀)-amido(alkyle en C₂-C₈)bétaïnes, les alkylamphodiacétates et leurs mélanges

14. Composition selon l'une quelconque des revendications précédentes, **caractérisée par le fait qu'**elle contient en outre au moins un additif de formulation choisi parmi les épaississants polymériques naturels ou synthétiques, anioniques, amphotères, zwittérioniques, non ioniques ou cationiques, associatifs ou non, les épaississants non-polymériques comme des acides ou des électrolytes, les agents nacrants, les agents opacifiants, les parfums, les huiles minérales, végétales et/ou synthétiques, les esters d'acides gras, les silicones volatiles ou non volatiles, organomodifiées ou non, les colorants, les agents conservateurs ou les agents de stabilisation du pH.

15. Composition selon l'une quelconque des revendications précédentes, **caractérisée par le fait qu'**elle se présente sous forme de shampooing, de produit pour le bain ou pour la douche, ou de produit démaquillant.

16. Composition selon la revendication 15, **caractérisée par le fait qu'**elle se présente sous forme de shampooing.

17. Procédé de traitement des matières kératiniques consistant à appliquer sur lesdites matières une composition selon l'une quelconque des revendications précédentes, et à procéder, après un éventuel temps de pose, au rinçage des matières kératiniques.

## Claims

1. Cosmetic composition comprising, in a cosmetically acceptable medium:
• at least one amphiphilic polymer chosen from branched block copolymers comprising:
(a) non-ionic units derived from at least one monomer chosen from C₁-C₂₀ alkyl (meth)-acrylates, N-mono (C₂-C₁₂ alkyl) (meth)-acrylamides and N,N-di(C₂-C₁₂ alkyl) (meth)-acrylamides;
(b) anionic units derived from at least one monomer chosen from acrylic acid and methacrylic acid; and
(c) polyfunctional units derived from at least one monomer comprising at least two polymerizable unsaturated functional groups;
• at least one cationic polymer; and
• at least 3% by weight, relative to the total weight of the composition, of at least one anionic surfactant.

2. Composition according to Claim 1, **characterized in that** the amphiphilic polymer chosen from the branched block copolymers has a structure composed of hydrophobic blocks attached to which, via polyfunctional units (c), are several more hydrophilic blocks.

3. Composition according to Claim 1 or 2, **characterized in that** the amphiphilic polymer has at least two glass transition temperatures (T_{g}), of which at least one is above ambient temperature (20°C) and the other is below ambient temperature.

4. Composition according to one of Claims 1 to 3, **characterized in that** the amphiphilic polymer is an anionic copolymer that mainly comprises:
(a) non-ionic units derived from butyl acrylate;
(b) anionic units derived from methacrylic acid and acrylic acid; and
(c) polyfunctional units derived from allyl methacrylate.

5. Composition according to Claim 4, **characterized in that** the amphiphilic polymer is mainly composed of:
(a) 27.5 mol% to 30.5 mol% of butyl acrylate;
(b) 26 mol% to 36 mol% of acrylic acid and 33.3 mol% to 45.3 mol% of methacrylic acid; and
(c) 0.48 mol% to 0.92 mol% of allyl methacrylate.

6. Composition according to any one of the preceding claims, **characterized in that** the concentration of the amphiphilic polymer is between 0.01% and 10% by weight relative to the total weight of the composition.

7. Composition according to any one of the preceding claims, **characterized in that** the cationic polymer or polymers are chosen from cellulose ethers comprising quaternary ammonium groups, cationic cyclopolymers based on dimethyldiallylammonium chloride, cationic polysaccharides such as guar gums modified by a 2,3-epoxypropyltrimethylammonium salt, quaternized polymers of vinylpyrrolidone and of vinylimidazole and polycondensates of poly(quaternary ammonium).

8. Composition according to any one of the preceding claims, **characterized in that** the cationic polymer or polymers are present in an amount between 0.001% and 10% by weight relative to the total weight of the composition.

9. Composition according to any one of the preceding claims, **characterized in that** the anionic surfactant or surfactants are chosen from alkyl sulphates, alkyl ether sulphates and alkyl ether carboxylates, and mixtures thereof.

10. Composition according to any one of the preceding claims, **characterized in that** the concentration of anionic surfactants is between 5 and 35% by weight relative to the total weight of the composition.

11. Composition according to any one of the preceding claims, **characterized in that** it additionally contains at least one surfactant chosen from non-ionic surfactants and amphoteric surfactants.

12. Composition according to Claim 11, **characterized in that** the non-ionic surfactant is a C₆-C₂₄ alkyl polyglycoside.

13. Composition according to Claim 11, **characterized in that** the amphoteric surfactant is chosen from (C₈-C₂₀ alkyl)betaines, (C₈-C₂₀ alkyl)amido(C₂-C₈ alkyl) betaines, alkyl amphodiacetates and mixtures thereof.

14. Composition according to any one of the preceding claims, **characterized in that** it additionally contains at least one formulation additive chosen from associative or non-associative, anionic, amphoteric, zwitterionic, non-ionic or cationic natural or synthetic polymeric thickeners, non-polymeric thickeners such as acids or electrolytes, pearlescent agents, opacifiers, fragrances, mineral, vegetable and/or synthetic oils, fatty acid esters, volatile or non-volatile silicones that may or may not be organo-modified, colorants, preservatives or pH stabilizers.

15. Composition according to any one of the preceding claims, **characterized in that** it is in the form of a shampoo, a bath or shower product, or a makeup-removing product.

16. Composition according to Claim 15, **characterized in that** it is in the form of a shampoo.

17. Method for treating keratinous substances that consists in applying a composition according to any one of the preceding claims to said substances and, after an optional leave-in time, in rinsing the keratinous substances.

## Patentansprüche

1. Kosmetische Zusammensetzung, die in einem kosmetisch akzeptablen Medium enthält:
• mindestens ein amphiphiles Polymer, das unter den verzweigten Blockcopolymeren ausgewählt ist, die enthalten
(a) nichtionische Einheiten, die von mindestens einem Monomer abgeleitet sind, das unter den Alkyl(C₁₋₂₀)(meth)-acrylaten, N-Monoalkyl(C₂₋₁₂)(meth)acrylamiden und N,N-Di(alkyl(C₂₋₁₂))(meth)acrylamiden ausgewählt ist,
(b) anionische Einheiten, die von mindestens einem Monomer abgeleitet sind, das unter Acrylsäure und Methacrylsäure ausgewählt ist, und
(c) polyfunktionelle Einheiten, die von mindestens einem Monomer stammen, das mindestens zwei polymerisierbare ungesättigte funktionelle Gruppen aufweist,
• mindestens ein kationisches Polymer, und
• mindestens 3 Gew.-% mindestens eines anionischen grenzflächenaktiven Stoffes, bezogen auf das Gesamtgewicht der Zusammensetzung.

2. Zusammensetzung nach Anspruch 1, **dadurch gekennzeichnet, dass** das amphiphile Polymer, das unter den verzweigten Blockcopolymeren ausgewählt ist, eine Struktur besitzt, die aus hydrophoben Blöcken besteht, an die über polyfunktionelle Einheiten (c) mehrere hydrophilere Blöcke gebunden sind.

3. Zusammensetzung nach Anspruch 1 oder 2, **dadurch gekennzeichnet, dass** das amphiphile Polymer mindestens zwei Glasübergangstemperaturen Tg besitzt, von denen mindestens eine über Raumtemperatur (20 °C) und die andere unter Raumtemperatur liegt.

4. Zusammensetzung nach einem der Ansprüche 1 bis 3, **dadurch gekennzeichnet, dass** das amphiphile Polymer ein anionisches Copolymer ist, das im Wesentlichen enthält:
(a) nichtionische Einheiten, die von Butylacrylat stammen,
(b) anionische Einheiten, die von Methacrylsäure und Acrylsäure stammen, und
(c) polyfunktionelle Einheiten, die von Allylmethacrylat stammen.

5. Zusammensetzung nach Anspruch 4, **dadurch gekennzeichnet, dass** das amphiphile Polymer im Wesentlichen besteht aus
(a) 27,5 bis 30,5 Mol-% Butylacrylat,
(b) 26 bis 36 Mol-% Acrylsäure und 33,3 bis 45,3 Mol-% Methacrylsäure, und
(c) 0,48 bis 0,92 Mol-% Allylmethacrylat.

6. Zusammensetzung nach einem der vorhergehenden Ansprüche, **dadurch gekennzeichnet, dass** die Konzentration des amphiphilen Polymers im Bereich von 0,01 bis 10 Gew.-%, bezogen auf das Gesamtgewicht der Zusammensetzung, liegt.

7. Zusammensetzung nach einem der vorhergehenden Ansprüche, **dadurch gekennzeichnet, dass** das oder die kationischen Polymere unter den Celluloseethern, die quartäre Ammoniumgruppen enthalten, kationischen Cyclopolymeren auf der Basis von Dimethyldiallylammoniumchlorid, kationischen Polysacchariden, wie mit einem 2,3-Epoxypropyltrimethylammoniumsalz modifizierten Guargummen, quaternisierten Polymeren von Vinylpyrrolidon und Vinylimidazol und Polykondensaten von quartärem Polyammonium ausgewählt sind.

8. Zusammensetzung nach einem der vorhergehenden Ansprüche, **dadurch gekennzeichnet, dass** das oder die kationischen Polymere in einer Menge von 0,001 bis 10 Gew.-%, bezogen auf das Gesamtgewicht der Zusammensetzung, enthalten sind.

9. Zusammensetzung nach einem der vorhergehenden Ansprüche, **dadurch gekennzeichnet, dass** der oder die anionischen grenzflächenaktiven Stoffe unter den Alkylsulfaten, Alkylethersulfaten und Alkylethercarboxylaten und deren Gemischen ausgewählt sind.

10. Zusammensetzung nach einem der vorhergehenden Ansprüche, **dadurch gekennzeichnet, dass** die Konzentration der anionischen grenzflächenaktiven Stoffe im Bereich von 5 bis 35 Gew.-%, bezogen auf das Gesamtgewicht der Zusammensetzung, liegt.

11. Zusammensetzung nach einem der vorhergehenden Ansprüche, **dadurch gekennzeichnet, dass** sie ferner mindestens einen grenzflächenaktiven Stoff enthält, der unter den nichtionischen grenzflächenaktiven Stoffen und amphoteren grenzflächenaktiven Stoffen ausgewählt ist.

12. Zusammensetzung nach Anspruch 11, **dadurch gekennzeichnet, dass** der nichtionische grenzflächenaktive Stoff ein Alkyl(C₆₋₂₄)-polyglycosid ist.

13. Zusammensetzung nach Anspruch 11, **dadurch gekennzeichnet, dass** der amphotere grenzflächenaktive Stoff unter den Alkyl-(C₈₋₂₀)betainen, Alkyl(C₈₋₂₀)amidoalkyl(C₂₋₈)betainen, Alkylamphodiacetaten und deren Gemischen ausgewählt ist.

14. Zusammensetzung nach einem der vorhergehenden Ansprüche, **dadurch gekennzeichnet, dass** sie ferner mindestens einen Formulierungshilfsstoff enthält, der unter den natürlichen oder synthetischen, anionischen, amphoteren, zwitterionischen, nichtionischen oder kationischen, assoziativen oder nichtassoziativen polymeren Verdickungsmitteln, nichtpolymeren Verdickungsmitteln, wie Säuren oder Elektrolyten, Perlglanzstoffen, Trübungsmitteln, Parfums, Mineralölen, pflanzlichen Ölen und/oder synthetischen Ölen, Fettsäureestern, flüchtigen oder nichtflüchtigen, organomodifizierten oder nichtorganomodifizierten Siliconen, Farbmitteln, Konservierungsmitteln oder pH-Stabilisatoren ausgewählt ist.

15. Zusammensetzung nach einem der vorhergehenden Ansprüche, **dadurch gekennzeichnet, dass** sie als Haarwaschmittel, als Produkt für das Bad oder die Dusche oder als Produkt zum Abschminken vorliegt.

16. Zusammensetzung nach Anspruch 15, **dadurch gekennzeichnet, dass** sie in Form eines Haarwaschmittels vorliegt.

17. Verfahren zur Behandlung von Keratinsubstanzen, das darin besteht, auf die Keratinsubstanzen eine Zusammensetzung nach einem der vorhergehenden Ansprüche aufzubringen und die Keratinsubstanzen nach einer gegebenenfalls abgewarteten Einwirkzeit zu spülen.
